(19) **Europäisches Patentamt / European Patent Office / Office européen des brevets**

(11) **EP 4 129 168 B1**

(12) **EUROPEAN PATENT SPECIFICATION**

(45) Date of publication and mention
of the grant of the patent:
**16.07.2025   Bulletin 2025/29**

(21) Application number: **21189442.3**

(22) Date of filing: **03.08.2021**

(51) International Patent Classification (IPC):
**A61B 5/06** (2006.01)   **A61B 5/053** (2021.01)

(52) Cooperative Patent Classification (CPC):
**A61B 5/068; A61B 5/053**

(54) **SYSTEM AND METHOD FOR AUTOMATIC LOCALIZATION OF THE SPATIAL POSITION OF ELECTRODES ON A CONDUCTIVE BODY**

SYSTEM UND VERFAHREN ZUR AUTOMATISCHEN LOKALISIERUNG DER RÄUMLICHEN LAGE VON ELEKTRODEN AUF EINEM LEITENDEN KÖRPER

SYSTÈME ET PROCÉDÉ DE LOCALISATION AUTOMATIQUE DE LA POSITION SPATIALE D'ÉLECTRODES SUR UN CORPS DE CONDUCTEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**08.02.2023   Bulletin 2023/06**

(73) Proprietor: **EP Solutions SA**
**1400 Yverdon-les-Bains (CH)**

(72) Inventors:
• **BRAUN, Fabian**
  **2000 Neuchâtel (CH)**
• **RAPIN, Michaël**
  **1530 Payerne (CH)**
• **WACKER, Josias**
  **4053 Basel (CH)**
• **CHÉTELAT, Olivier**
  **1588 Cudrefin (CH)**

(74) Representative: **Studio Torta S.p.A.**
**Via Viotti, 9**
**10121 Torino (IT)**

(56) References cited:
**WO-A1-2020/212527**

**Description**

TECHNICAL FIELD

The present invention relates to a system and method for automatic localization of the spatial position of electrodes on a conductive body, in particular in the field of physiological measures.

BACKGROUND ART

As it is known, in the field of physiological signal measurements there are several applications wherein sensors and/or electrodes are placed on a surface of a conductive body and their spatial location on this surface has to be known, e.g., as contextual information to correctly exploit measurement of the physiological signals.

Sensors could be for instance a stethoscope and the contextual information its spatial position on the skin of the thorax. Electrodes could be for instance those used in bioimpedance and/or biopotential measurements such as electrocardiography (ECG), electromyography (EMG), ECG imaging (ECGI), electrical impedance tomography (EIT), or electroencephalography (EEG), and the contextual information their spatial position on the surface of the body.

A harmless and low-cost method for 2D-localization of sensors and/or electrodes is photographic imaging, e.g., performed around the torso of a subject with the sensors/electrodes applied thereto. For this analysis, 3D cameras, 3D depth cameras, or moving cameras in combination with advanced image processing algorithms are generally used.

For example, optical solutions of this kind are disclosed in:

E. A. Perez-Alday et al., "Torso geometry reconstruction and body surface electrode localization using three-dimensional photography," Journal of Electrocardiology, vol. 51, no. 1, pp. 60-67, Jan. 2018;
E. A. Perez-Alday et al., "Torso mesh and body surface electrodes position reconstruction using a Kinect camera," Journal of Electrocardiology, vol. 51, no. 1, pp. e4-e5, Jan. 2018;
R. N. Ghanem, C. Ramanathan, Ping Jia, and Y. Rudy, "Heart-surface reconstruction and ECG electrodes localization using fluoroscopy, epipolar geometry and stereovision: application to noninvasive imaging of cardiac electrical activity," IEEE Transactions on Medical Imaging, vol. 22, no. 10, pp. 1307-1318, Oct. 2003.

With such optical methods, the error between the optical localization and a reference localization (achieved e.g., via Computed Tomography - CT, and Magnetic Resonance Imaging - MRI) may be for example about 1 cm.

Since patients are typically in a supine position, a problem of such optical methods is the determination of the location of sensors/electrodes on the back of the subject. This problem could be tackled by use of a transparent bed (as disclosed e.g. in RU 2 733 470 C1), or of a soft material in which the patient's back may leave an imprint that is recorded with 3D photo-scanning and computationally combined with 3D images of the front of the patient (as disclosed e.g. in WO 2019/235969 A1).

The optical detection of sensors/electrodes requires in any case dedicated setups and apparatuses (e.g., the cameras and the corresponding accessories, including a transparent bed or a soft material covering the bed).

Other known solutions for the localization of electrodes require the use of additional instruments to localize the electrodes, e.g. ultra-sound transducers (as disclosed e.g. in WO 2018/218244 A1), or radiological imaging devices (as disclosed e.g. in WO 2011/159688 A2); or imply the use of invasive (catheter-based) methods to determine the 3D-position of probes or electrodes inside the body (as disclosed e.g. in US 5297549 A, in relation to the generation of a three-dimensional electrical map of the interior of the heart chamber).

For the particular case of ECGI measurements, currently, MRI or CT scans are also used to determine the location of the measurement electrodes. These scans are costly, potentially harmful, and logistically challenging, since they require the coordination between different departments in a hospital; the latter is because, for example, ECGI examination has to be scheduled together with the MRI or CT-scan session (which has to be repeated at each visit/measurement); this is an important barrier for acceptance in clinical routine due to logistical difficulties.

All these known solutions therefore require dedicated setups and apparatuses or undesired invasive interventions inside the body of the subject.

WO 2020/212527 A1 discloses a method of computing a dielectric map, comprising exciting at least one pair of electrodes according to an excitation scheme, the at least one pair of electrodes comprising at least one pair of in-body (intra-body) electrodes located inside of the examined living body, measuring and recording voltages developing on the in-body electrodes during the excitation according to the excitation scheme, solving an inverse problem to derive a 3D dielectric map from the recorded voltages and optionally providing a 3D image of the body tissues based on the 3D dielectric map. Methods are also disclosed that combine intrabody electrodes and surface electrodes secured to the body or use only surface electrodes.

## DISCLOSURE OF INVENTION

It is an object of the present solution to provide a system and method for the automatic localization of the spatial position of electrodes on a conductive body, in particular of a living being, which allow to solve at least partly the above-mentioned inconveniences.

According to the present solution, an automatic electrode localization system and method are therefore provided, according to the appended claims.

## BRIEF DESCRIPTION OF THE DRAWINGS

A non-limiting embodiment of the present invention will be described by way of example with reference to the accompanying drawings, in which:

- Figure 1 is a schematic representation of a conductive body of a living being with electrodes placed on a surface thereof and of an automatic electrode localization system coupled to the conductive body, according to one embodiment of the present solution;

- Figures 2 and 3 are diagrammatic flow diagrams of operations performed by the automatic electrode localization system of Figure 1;

- Figure 4 is a schematic depiction of isopotential lines resulting from the injection of a current between two or more of the electrodes of the automatic electrode localization system of Figure 1;

- Figures 5A-5B show plots of localization probability functions related to the operation of the automatic electrode localization system of Figure 1; and

- Figure 6 shows a model of a human thorax whereon electrodes of the automatic electrode localization system of Figure 1 have been applied.

## BEST MODE FOR CARRYING OUT THE INVENTION

As will be discussed in more details in the following, according to the present solution the spatial positions of electrodes, in particular so-called measurement electrodes, on the surface of a conductive body are determined via impedimetric measurements, via processing of voltage potentials measured at these measurement electrodes and resulting from electrical currents injected at other electrodes, in particular so-called landmark electrodes, having a known spatial position on the same surface of the conductive body.

It is noted that, according to another possible embodiment, due to the reciprocal nature of the impedance measurements, the electrical current may be injected via at least some of the measurement electrodes and the voltage potentials may be measured at the landmark electrodes and/or at other measuring electrodes (not used for the current injections).

It is also noted that a sensor and/or electrode to be localized (e.g. to be used for physiological measurements) may be combined with a measurement electrode, in case the sensor or electrode to be localized does not include or coincide with the same measurement electrode.

As will also be discussed in detail in the following, the present solution uses a 3D (three-dimensional) model of the conductive body, in particular an electrical impedance model of the conductive body, in order to perform the electrode localization by means of a joint, or combined, processing of voltages measured at the electrodes and of body surface potentials (BSPs) estimated via the electrical impedance model.

The 3D-model of the conductive body may also include information about the electrical conductivity of tissues and/or the morphology of internal structures of the conductive body (e.g., organs, in the case of the human thorax).

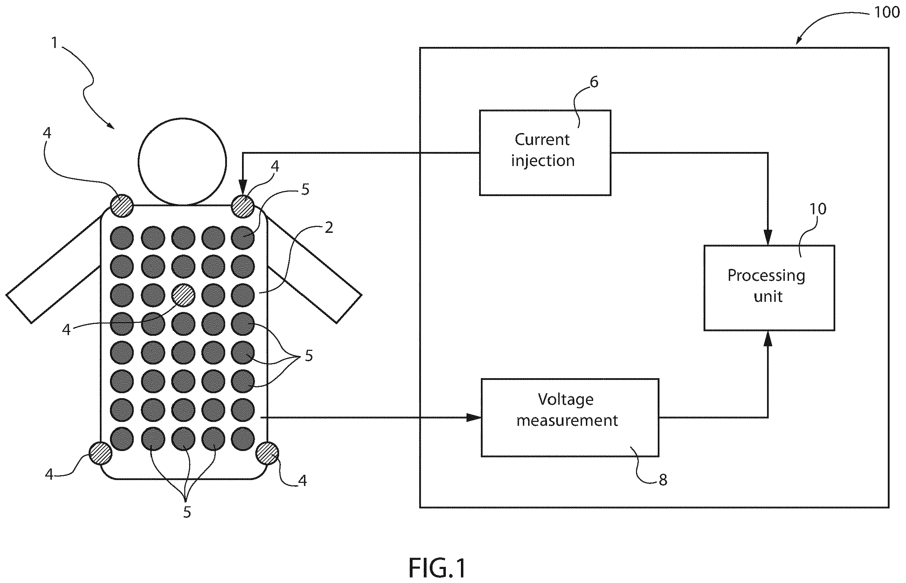

Figure 1 schematically shows a system 1 for the automatic localization of the spatial position of electrodes on a conductive body 2 (in this exemplary depiction being the torso of a human being), according to an embodiment of the present solution.

The system 1 comprises first electrodes, in particular landmark electrodes (denoted in the following as LEs), 4 that can be placed in a repeatable manner at definite and identifiable spatial locations on the surface of the conductive body 2, for instance by means of: anatomical landmarks or points (e.g., the clavicula, sternum, hipbone, etc.); and/or points temporarily or permanently marked (e.g., via tattoos, stickers, or any other suitable marker); and/or biopotential measurements (e.g., measurements of ECG signals detected at the same spatial locations), bioimpedance measurements (EIT), sound measurements or similar.

In the example shown in Figure 1, the first electrodes 4 are placed at the shoulders, hips and at the xiphoid process of the sternum of the subject (the number of first electrodes can, however, clearly vary, according to the practical implementation, e.g., generally from a minimum number of three).

The system 1 also includes second electrodes, in particular measurement electrodes (denoted in the following as MEs) 5, which are placed at unknown spatial locations (x,y,z) on the surface of the conductive body 2 and are therefore to be localized by the same system 1.

These second electrodes 5 may be associated, in use, with sensors or electrodes to be localized for physiological measurement of, for instance, biopotentials (ECG or ECGI), bioimpedance (EIT), sounds, photoplethysmography (PPG), etc.; the second electrodes 5 can also coincide with the same electrodes used for the physiological measurements.

The system 1 further comprises: a current injection module 6, which is configured to inject a measuring current (which is not harmful for the body, e.g., of a few tens of microamperes at a frequency, for instance, in the range of 100 Hz to 100 kHz) at the electrodes, for example at the first electrodes 4; and a voltage measuring module 8, which is configured to measure resulting voltages at the electrodes, for example at the second electrodes 5 (or also at the first electrodes 4).

The current injection by the current injection module 6 is a bipolar injection of current between the electrodes, for example between all possible pairs of first electrodes 4 (however, in general, any of the electrodes, in particular any of the first electrodes 4, second electrodes 5 or any possible combination of the same electrodes can be considered for the current injection); and the voltages are measured by the voltage measuring module 8 at the surface of the conductive body 2, e.g., as bipolar voltages between the electrodes, for example between all possible pairs of second electrodes 5 (however, also in this case, any of the electrodes, first electrodes 4 or second electrodes 5, or any combination thereof can be considered for the voltage measurement).

In particular, as previously noted, as an alternative, and again considering the reciprocity theorem, the current injections can be performed at some or several of the MEs (e.g., between pairs of the same MEs) and the resulting measured voltages can be detected at some or several of the LEs. Any suitable combination can also be envisaged, e.g., with current injections being performed both with LEs and MEs and the voltage measurements being also performed both with respective MEs and LEs.

The system 1 further comprises a processing unit 10, e.g., including a microprocessor, a microcontroller or any suitable computing unit, which is operatively coupled to the voltage measuring module 8 in order to receive the measured voltages, i.e., the voltages detected at some of the first electrodes 4 and second electrodes 5 as a result of the injection of currents at others of the first electrodes 4 and second electrodes 5.

The processing unit 10 is configured to implement a dedicated electrode localization algorithm to localize the electrodes (i.e., to determine their corresponding spatial location on the surface of the conductive body 2) via a joint processing of the voltages measured at the same electrodes and of information extracted from a three-dimensional (3D) electrical impedance model of the conductive body 2.

The processing unit 10 is configured to output the determined spatial locations of the electrodes (e.g., given as 3D-coordinates x,y,z on the surface of the conductive body 2 or as parameters u,v of the same surface parametrized in two dimensions - 2D); according to an aspect of the present solution, the processing unit 10 is further configured to output a localization reliability index for each performed localization (as will be discussed in details in the following).

In particular, the electrical impedance model of the conductive body 2 is a three-dimensional representation (e.g., via a finite element model) of the conductive body 2, with information about the electrical properties (including electrical conductivity and permittivity) suitable for the calculation of BSPs, that are estimated voltage potentials (at corresponding spatial locations in the model) resulting from the simulated injection of currents at the electrodes.

The electrical impedance model may further include 3D representations of internal structures (e.g., organs such as heart, liver, lungs, aorta, etc.) together with respective electrical properties, such as electrical conductivity and permittivity, of each of these internal structures.

It is noted that the electrical impedance model of the conductive body 2 can be generated via any suitable known method, at least in part by the same processing unit 10 or by any other external computing device (here not shown) cooperating with the processing unit 10; a predefined 3D electrical impedance model of the conductive body 2 may also be used, e.g., stored in a memory (here not shown) coupled to the processing unit 10.

For example (as disclosed e.g. in Lenchik, Leon, et al. "Automated segmentation of tissues using CT and MRI: a systematic review", Academic radiology 26.12 (2019): 1695-1706), the 3D electrical impedance model can be generated from an MRI or CT scan (or any other 3D imaging suitable to obtain body morphology) of the conductive body 2, using this scan to establish an individual electrical impedance model.

Common software tools for the generation of such individual electrical impedance models of the conductive body 2 are for example Simpleware (https:// www.synopsys.com/simpleware.html); Seg3D (https:// www.sci.utah.edu/cibc-software/seg3d.html); 3D Slicer (https://www.slicer.org/).

The electrical impedance model can also be generated from 3D photography or a depth camera, as disclosed e.g. in:

E. A. Perez-Alday et al., "Torso geometry reconstruction and body surface electrode localization using three-dimensional photography", Journal of Electrocardiology, vol. 51, no. 1, pp. 60-67, Jan. 2018;
E. A. Perez-Alday et al., "Torso mesh and body surface electrodes position reconstruction using a Kinect camera", Journal of Electrocardiology, vol. 51, no. 1, pp. e4-e5, Jan. 2018.

The computation of BSPs, given an electrical impedance model and a set of current injecting electrodes is also well known in the art in various domains, such as Electrical Impedance Tomography (EIT), as discussed for example in D. Holder, ed., "Electrical impedance tomography: methods, history, and applications", Institute of Physics Publishing, Bristol, UK, 2005.

Reconstruction software like EIDORS (http://eidors3d.sourceforge.net/) or COMSOL Multiphysics (https://www.comsol.com/) or any other suitable software can be used for this purpose.

In particular, the processing unit 10 is configured to: identify the first electrodes 4, with known positions, in the electrical impedance model (e.g., based on known anatomical locations or reference electrical signals; alternatively, in case the model is generated via a previous MRI or CT-scan or any other imaging technique, the position of the first electrodes 4 can also be imaged and thereby identified by the processing unit 10); and simulate the current injections at the same first electrodes 4 (as discussed, between any suitable combination of the first electrodes 4) to estimate, for each current injection, a resulting expected or estimated BSP distribution.

The processing unit 10 is then configured to compare, for each second electrode 5, the estimated BSP with the voltage measured at the same second electrode 5 (for all the considered current injections), in order to localize its spatial position.

In particular, each second electrode 5 is localized by determining the spatial position on the surface of the conductive body 2 that provides an estimated voltage potential that most closely corresponds to (i.e., is closest to) the voltage measured at the same second electrode 5, considering all current injections.

According to a possible embodiment, the same processing unit 10 may control the current injection module 6 and the voltage measuring module 8; in particular, the current injection module 6, the voltage measuring module 8 and the processing unit 10 may be integrated in a single localization device 100, that can be coupled to the conductive body 2.

As an alternative, at least part of the current injection module 6 and/or the voltage measuring module 8 can be provided separately from the processing unit 10, e.g., being integrated in sensor units coupled to the body 2 (and including corresponding electrodes).

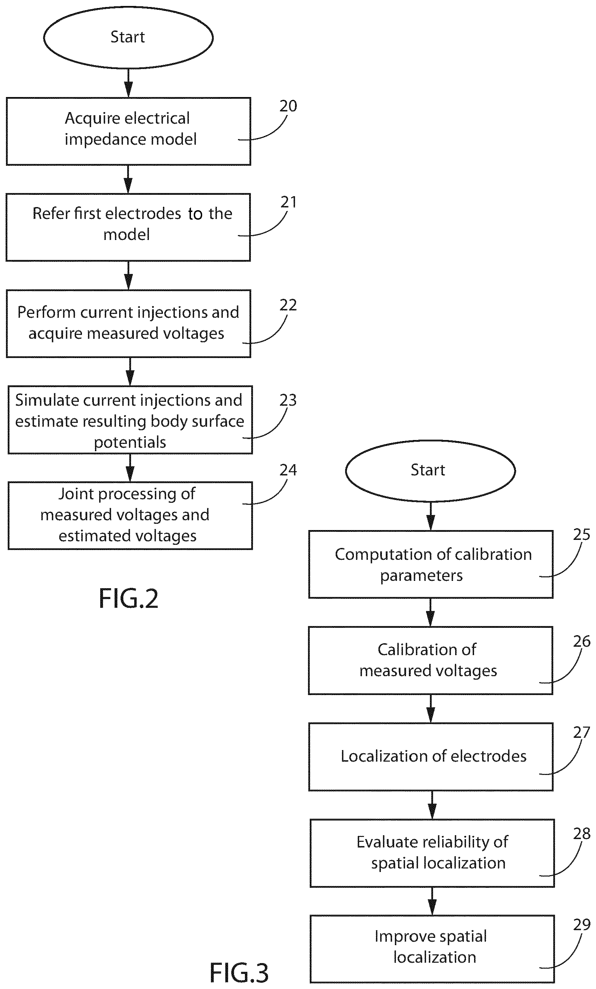

Referring to Figure 2, the operation of the system 1 and in particular the processing steps performed by the processing unit 10 for implementing the electrode localization are now discussed in more details (it is noted that the steps discussed in the following can be applied either only once or multiple consecutive times, i.e., in an iterative manner, in order to improve the localization accuracy).

In a first step 20, the processing unit 10 is configured to acquire the electrical impedance model of the conductive body 2, which will be used to estimate the BSPs resulting from the simulated current injections.

As previously discussed, this electrical impedance model can be received from an external device, e.g., based on an MRI or CT scan of the conductive body 2, which can possibly be further processed by the same processing unit 10; or else it can be a predefined model, e.g., stored in a memory coupled to the same processing unit 10.

At step 21, the processing unit 10 refers the first electrodes 4 to the electrical impedance model.

Then, at step 22, current injections are performed, e.g., between two or more of the first electrodes 4, and the resulting voltages are acquired, by the voltage measuring module 8, at the surface of the conductive body 2, e.g., as bipolar voltages between all possible pairs of electrodes.

As shown at step 23, the same current injections are simulated in the electrical impedance model, so as to estimate the resulting BSPs.

At step 24, joint processing of the measured voltages and the estimated voltage potentials is then performed, in order to determine the electrodes' spatial locations.

Referring also to Figure 3, according to a possible embodiment, this step of joint processing may include a preliminary calibration procedure, for correction of the measured voltages, based on the estimated voltage potentials obtained by the electrical impedance model (it is noted, however, that this preliminary calibration step may be omitted, at least in some applications).

In particular, for each available current injection i (denoted in the following by an index i) a corresponding calibration function $Cal_i$ can be used to determine calibrated voltages $\bar{u}_{i,m}$ from the voltages $u_{i,m}$ measured (for the related current injection i) at any respective electrode m:

$$\bar{u}_{i,m} = Cal_i(u_{i,m}).$$

As shown at step 25, calibration parameters for the calibration function $Cal_i$ can be obtained, for each available current injection, by calibrating measured voltages $u_{i,k}$ against estimated or modelled voltages $\hat{u}_{i,k}$ obtained from the model, where the measured voltages $u_{i,k}$ are in this case limited to a set of electrodes (k being an index iterating over all electrodes in this set) with known position (in particular, being first electrodes 4, LEs, and possibly, in further iterations of the procedure, optionally also second electrodes, MEs, localized, as will be discussed, with a sufficiently high reliability).

For instance, for each current injection i individually, corresponding potential offset $v_0(i)$ and gain $g(i)$ in voltage measurements can be determined with a least mean square (LMS) algorithm finding the corresponding values $v_0$ and $g$ minimizing the square error:

$$e^2 = \sum_k (v_0(i) + g(i)u_{i,k} - \hat{u}_{i,k})^2 \,.$$

As shown at step 26, the calibration parameters are then applied to all voltages $u_{i,m}$, measured for the related current injection i at any electrode m (in particular, of the second electrodes 5), to obtain the corresponding calibrated voltages $\overline{u}_{i,m}$ by means of the corresponding calibration function $Cal_i$.

In a possible example, a linear regression calibration function $Cal_i$ (with calibration parameters $v_0(i)$ and $g(i)$) can be used to determine the calibrated voltages $\overline{u}_{i,m}$, according to the expression:

$$\overline{u}_{i,m} = Cal_i(u_{i,m}) = v_0(i) + g(i) \cdot u_{i,m} \,.$$

It is noted, however, that the above linear regression is only an example and that other calibration functions $Cal_i$ could be used (possibly based on different and further calibration parameters).

It is also noted that a goodness of fit of each calibration function (e.g., being the inverse of the above defined square error $e^2$) can be included in a weighting factor $w_i$, which regulates the individual contribution of each current injection i to the electrode localization.

As shown at step 27, each respective electrode m, in particular of the second electrodes 5, is then localized by finding the spatial position on the conductive body 2 (e.g. given as 3D-coordinates x,y,z on the surface of the same conductive body 2) that provides the modelled voltage $\hat{u}_{i,m}(x,y,z)$ that is closest to the corresponding calibrated voltage $\overline{u}_{i,m}$ at that electrode (it is noted, however, that the measured voltage $u_{i,m}$ could be used instead), taking into account all current injections i.

For each current injection i and corresponding voltage $u_{i,m}$ measured at a respective electrode m, a localization function is determined on the electrical impedance model that represents a group of possible spatial locations corresponding to that measured voltage $u_{i,m}$. The location of the respective electrode m is then determined via a joint processing of all the corresponding localization functions (and groups of possible locations) obtained for all the current injections i.

The above localization functions can be of various types, for example being based on isopotential lines or probability functions.

In more details, according to a first possible embodiment, the spatial position of each respective electrode m can be determined via processing of the intersections of all the related isopotential lines obtained for each current injection i.

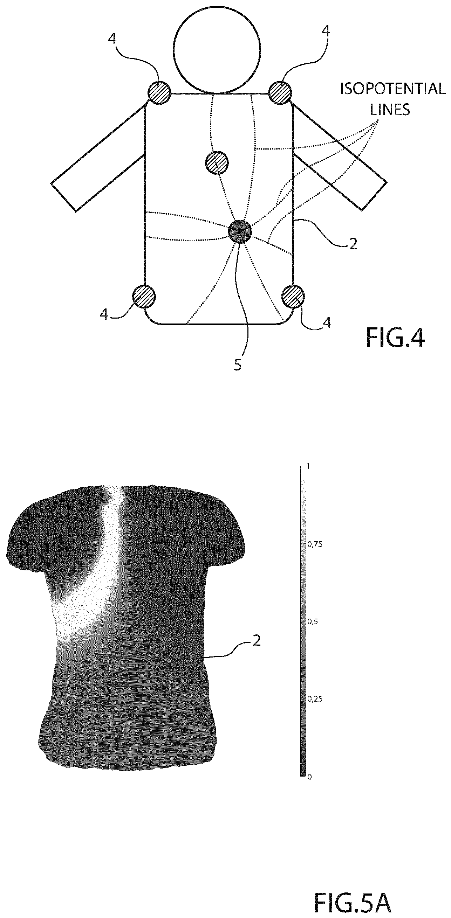

In particular, considering each respective electrode m, for each current injection i and corresponding calibrated voltage $\overline{u}_{i,m}$ (or measured voltage $u_{i,m}$), an isopotential line is obtained on the electrical impedance model (see the dotted lines in Figure 4, relating to just one of the second electrodes 5, for clarity of depiction), indicative of all possible locations on the surface of the conductive body 2 matching that calibrated voltage $\overline{u}_{i,m}$. Having N current injections leads to N corresponding isopotential lines, of which all possible pairs cross in a number $N_x$ of intersections equal to:

$$N_x = \binom{N}{2} = \frac{N!}{2(N-2)!},$$

leading to $N_x$ predicted locations for the considered electrode.

The final spatial location for the respective electrode may be determined for example via a weighted mean or weighted average of all isopotential line intersections.

Weighting factors $w_i$ (one value for each current injection i and/or for each of the $N_x$ isopotential intersections) used for the above weighted mean or average can take into account the goodness of fit of the respective calibration (as discussed above), and/or a sensitivity to electrode displacement (as will be discussed below), and/or a sensitivity to internal conductivity changes (as will also be discussed below).

In a second possible embodiment, the spatial position of each respective electrode m (e.g., of each second electrode 5, ME) is determined via an averaged localization probability.

In more details, again considering each respective electrode m, for each current injection i a difference function is computed between the calibrated voltage $\overline{u}_{i,m}$ (or the measured voltage $u_{i,m}$) at that electrode and the modelled voltage

$u_i(x,y,z)$ resulting from the BSP associated with the same current injection i.

This difference function, denoted as $\Delta\Phi_{i,m}(x,y,z)$, can be expressed as an absolute difference:

$$\Delta\Phi_{i,m}(x,y,z) = \left|\bar{u}_{i,m} - \hat{u}_i(x,y,z)\right|;$$

a relative difference:

$$\Delta\Phi_{i,m}(x,y,z) = \left|\bar{u}_{i,m} - \hat{u}_i(x,y,z)\right|/\bar{u}_{i,m};$$

or any other suitable function:

$$\Delta\Phi_{i,m}(x,y,z) = f\left(\bar{u}_{i,m}; \hat{u}_i(x,y,z)\right).$$

A localization probability function $f_T$, e.g.:

$$f_T: \quad \Delta\Phi_{i,m}(x,y,z) \rightarrow \exp(-\Delta\Phi_{i,m}(x,y,z)).$$

is then used to convert the above difference function $\Delta\Phi_{i,m}(x,y,z)$ to a probability in the range ([0, 1]), i.e., forcing large errors to tend to a probability of zero and small errors to a probability of one.

The probability function $f_T(\Delta\Phi_{i,m}(x,y,z))$ is therefore indicative of a probability of localization of the respective electrode m at any spatial location $(x,y,z)$ on the surface of the conductive body 2.

In this respect, Figure 5A shows an exemplary localization probability function for one of the second electrodes 5 and considering one of the possible current injections; the area with a resulting highest localization probability is shown with brightest color.

An average localization probability can then be computed by a weighted averaging of the localization probability functions $f_T(\Delta\Phi_{i,m}(x,y,z))$ of all N current injections; this weighted averaging may use the same weighting factors $w_i$ as discussed above for the first embodiment.

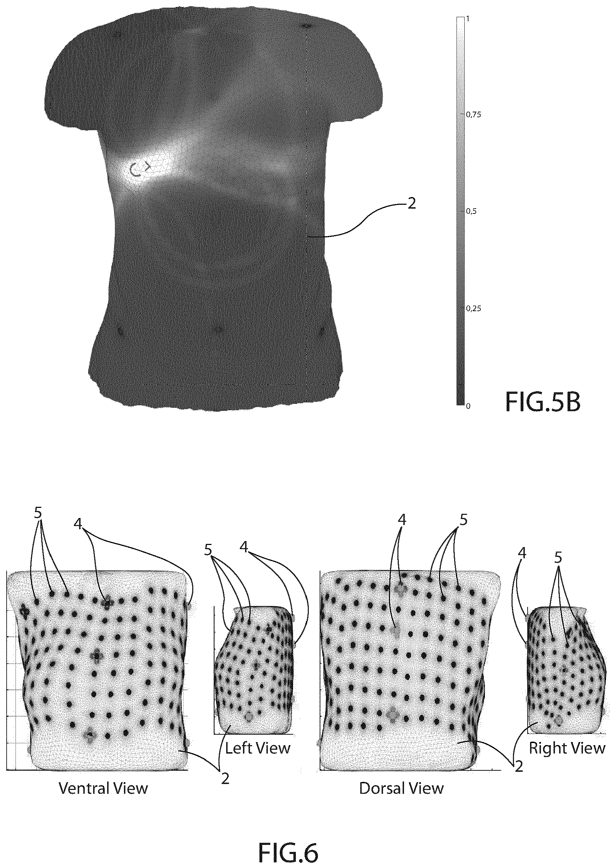

Figure 5B shows an exemplary result of the above averaging of localization probability functions $f_T(\Delta\Phi_{i,m}(x,y,z))$ for one of the second electrodes 5; the area with a highest average probability is again shown with brightest color.

The spatial position of each second electrode 5 in this case can be localized at the position where the average localization probability has its maximum.

As shown at step 28 (see again Figure 3), the processing unit 10 can be further configured to evaluate a reliability associated with the spatial localization of electrodes, computing a localization reliability index (LRI). For example, an LRI of 0 may correspond to a worst possible localization and an LRI of 1 to a best possible localization.

As an example, this LRI can be used in a final application of the electrodes (e.g., for ECGI or EIT or other physiological measurements) to exclude or attenuate the influence of certain electrodes with bad or unreliable localization in any further processing.

In more details, considering the first embodiment discussed above, the processing unit 10 may be configured to determine the LRI as a function of the dispersion of the isopotential line intersections (e.g., as the standard deviation of all - or of a subset of - isopotential line intersections), or as the average or median of the angles between each pair of isopotential lines at their intersection.

Considering the second embodiment discussed above, the processing unit 10 may be configured to determine the LRI as a function of the value of the maximum average localization probability or the steepness/width/skewness of the average localization probability at the position of the corresponding electrode.

Moreover, according to a further aspect of the present solution, as shown at step 29, the processing unit 10 may be further configured to improve the localized positions of the electrodes.

For example, this improvement may be implemented by considering geometrical a-priori knowledge and/or constraints such as minimal and maximal distances between electrodes and/or information about groups of electrodes connected together, e.g., in strips or patches.

For instance, two electrodes connected together at a fixed distance may be localized simultaneously by determining their most likely positions while considering the constraint imposed by their fixed distance. This can be extended to more electrodes, with different and more complicated geometric constraints (e.g., multiple electrodes connected together in patches or strips).

The electrodes, or electrode strips or electrode patches including the landmark electrodes, may also contain a crosshair cursor to help the placement accuracy and/or a crosshair marker to allow a medical operator to read any placement inaccuracy and input it to the processing unit 10 to be considered by the localization algorithm for an improved localization. The distance between electrodes can also be measured (e.g., with a measuring tape) by the medical operator and input to the same processing unit 10, to similarly be considered by the localization algorithm.

The localized electrode positions can further be adjusted by using information on other measurements, such as ECG-derived surface potentials or IMU (Inertial Measuring Unit) data. For instance, the spatial position of a ME can be further corrected by considering differences in ECG-derived surface potentials between the same electrode to be localized and neighboring electrodes (MEs or LEs), or by considering IMU-derived measuring electrode orientation and comparing it to an expected orientation in the model and to the orientation of neighboring electrodes.

The present Applicant has tested the reliability and accuracy of the proposed solution by means of several tests and numerical simulations.

In particular, for an exemplary application to ECGI measurements, various 3D models of adult human thoraxes were generated from CT and/or MRI images and used for simulating bio-impedance and bio-potential measurements.

Figure 6 refers, in this respect, to an exemplary 3D model of human thorax of a human adult with 224 electrodes arranged in 28 vertical strips, of which 10 electrodes are used as LEs (highlighted dots) and the remaining 214 electrodes as MEs (the electrodes have in this example a diameter of 1 cm).

In the example, the injected current between the electrodes was 10 $\mu$A, with a number of bipolar injections equal to:

$$\binom{10}{2} = \frac{10!}{2!(10-2)!} = 45\,.$$

The advantages of the discussed solution will be clear from the foregoing description.

In any case, it is underlined again that, compared to known solutions, the above discussed automatic localization system 1 does not require additional devices and setups for the spatial localization of sensors and/or electrodes. This allows cheaper equipment and procedures, and makes the localization possible at any time, even in a continuous manner and during concurrent physiological measurements.

In particular, if the sensing elements used for physiological measurements are, or include, electrodes (e.g., for ECGI measurements), they may also share the function of measurement electrodes (so that no hardware modifications are required by the present localization system).

Clearly, changes may be made to what is described herein without, however, departing from the scope of protection as defined in the accompanying claims.

In particular, it is underlined again that the discussed localization algorithm can also be applied to localize the LEs. This localization can be used to compute a LRI for the LEs and reject (or attenuate the influence of) some of the same electrodes in further iterations of the localization algorithm. This may be useful, e.g., to discard LEs, which were not accurately placed on the anatomical or reference points on the surface of the conductive body 2.

Moreover, it is noted that, once localized with sufficient reliability, a ME can also be used as LE (i.e., as one of the first electrodes 4) in subsequent iterations of the localization algorithm.

In particular, MEs can optionally also play the role of LEs, once their spatial location is known, to further improve the accuracy of localization of the other MEs and/or to deduce the corresponding LRI.

It is again noted that the above-discussed weighting factors $w_i$ can also be a function of the sensitivity with respect to displacements of the first electrodes 4 and/or of the sensitivity with respect to internal conductivity changes.

In particular, in a possible pre-processing step, the electrical impedance model can be used to compute the sensitivity at any location x,y,z on the surface of the conductive body 2 to the displacement of the first electrodes 4. This may result in a map for the whole conductive body 2 indicating, for every first electrode 4, an expected localization error. Combining this for all the first electrodes 4 active during a current injection results in a respective cumulative error for each of the N current injections. This cumulative error may then be included in the weighting factors $w_i$ by penalizing (for $w_i$ close to zero) injections with high errors and favoring (for $w_i$ close to one) injections with low errors due to electrode displacement.

Similarly, the weighting factors $w_i$ can be adapted such that those injections with lowest sensitivity to internal conductivity uncertainties are favored over injections with higher sensitivity to internal conductivity uncertainties.

It is also noted that the current injections and resulting voltage measurements performed for the impedance procedure can be restricted to specific timepoints or time interval in the cardiac and/or respiratory cycle. This restriction can either be performed in real-time (by limiting measurements to specific timepoints in the cardio-respiratory cycles) or in a post-processing step (by rejecting certain measurements). This cardiorespiratory "gating" may be performed by assessing the cardiac and respiratory cycle via ECG and/or bio-impedance and/or IMU measurements and/or PPG and/or EMG and/or any other suitable method to assess cardiorespiratory events. In a corresponding manner, measurements with too much body movement, coughing, or muscular contractions, etc. can be rejected.

Moreover, the properties of the electrical impedance model of the conductive body 2 can be updated iteratively by optimizing, e.g., for a best possible goodness of fit and/or for a highest possible LRI of each ME. This may allow to correct the electrical properties of the model to be closer to reality (e.g., in case the measurements are performed on a body of partially unknown conductivity).

**Claims**

1. A system (1) for the automatic localization of electrode spatial position on the surface of a conductive body (2), comprising a processing unit (10) configured to:

   in response to bipolar electrical current injections at first electrodes (4) on the surface of the conductive body (2), acquire voltages measured at second electrodes (5) on the surface of the conductive body (2);
   using an electrical impedance model of the conductive body (2), where the electrical current injections at the first electrodes (4) are simulated, estimate, for each electrical current injection, a resulting body surface potential on the conductive body (2);
   perform a combined processing of the voltages measured at the second electrodes (5) and of the estimated body surface potential, in order to determine the locations of the second electrodes (5) on the surface of the conductive body (2);
   wherein the processing unit (10) is configured to determine the location of a respective electrode (m) as the spatial position on the surface of the conductive body (2) that provides in the estimated body surface potential given by the electrical impedance model a modelled voltage ($\hat{u}_{i,m}$), which most closely corresponds to the voltage ($u_{i,m}$) measured at the respective electrode (m), considering all current injections (i),
   and wherein the processing unit (10) is configured to:

      determine, for each current injection (i) and corresponding voltage ($u_{i,m}$) measured at the respective electrode (m), a localization function on the electrical impedance model that represents a group of possible locations corresponding to that measured voltage ($u_{i,m}$); and
      determine the location of the respective electrode (m) via a combined processing of all the corresponding localization functions obtained for all the current injections (i).

2. The system according to claim 1, wherein the processing unit (10) is configured to:

   obtain, for each current injection (i) an isopotential line on the electrical impedance model based on the corresponding voltage ($u_{i,m}$) measured at the respective electrode (m), the isopotential line being indicative of all possible locations on the surface of the conductive body (2) matching that measured voltage ($u_{i,m}$); and
   determine the location of the respective electrode (m) based on a weighted mean or average of the intersections of all isopotential lines obtained for all the current injections.

3. The system according to claim 2, wherein the processing unit (10) is configured to:

   compute, for each current injection (i), a difference function ($\Delta\Phi_{i,m}(x,y,z)$) based on a difference between the corresponding voltage ($u_{i,m}$) measured at the respective electrode (m) and the modelled voltage $\hat{\mu}_{i,m}$ on the surface of the conductive body (2) derived from the electrical impedance model;
   determine, based on the difference function ($\Delta\Phi_i(x,y,z)$), a respective probability function ($f_T(\Delta\Phi_i(x,y,z))$) indicative of a probability of localization of the respective electrode (m) at any spatial location (x,y,z) on the surface of the conductive body (2);
   compute an average localization probability by averaging of the probability functions ($f_T(\Delta\Phi_i(x,y,z))$) of all the current injections; and
   determine the location of the respective electrode (m) based on the computed average localization probability.

4. The system according to any of the preceding claims, wherein the processing unit (10) is configured to assign to each localization function and each corresponding current injection (i) a respective weighting factor ($w_i$) in the combined processing, the weighting factor ($w_i$) regulating the individual contribution of each current injection (i) to the electrode localization and being indicative of a localization reliability associated with each current injection (i).

5. The system according to claim 4, wherein the processing unit (10) is configured to:

   perform, for each current injection (i), a calibration procedure for correction of the measured voltages ($u_{i,m}$) via a

calibration function ($Cal_i$) based on the modelled voltages ($\hat{u}_{i,m}$) estimated by the electrical impedance model; determine a goodness of fit of each calibration function ($Cal_i$); determine, based on the related goodness of fit, the weighting factor ($w_i$) which regulates the individual contribution of each current injection (i) to the electrode localization.

6. The system according to any of the preceding claims, wherein the processing unit (10) is configured to:

compute, for each injection (i), calibration parameters for correction of the measured voltages ($u_{i,m}$), by calibrating voltages ($u_{i,k}$) measured at a set of electrodes with known or identifiable positions against modelled voltages ($\hat{u}_{i,k}$) estimated from the electrical impedance model for the same set of electrodes with known or identifiable positions; and correct all measured voltages ($u_{i,m}$) for the related current injection (i) at any respective electrode (m) to obtain corresponding calibrated voltages ($\overline{u}_{i,m}$), by means of a calibration function ($Cal_i$) and the computed calibration parameters.

7. The system according to any of the preceding claims, wherein the processing unit (10) is further configured to evaluate a reliability associated with the spatial localization of each second electrode (5), computing a corresponding localization reliability index (LRI).

8. The system according to any of the preceding claims, wherein the first electrodes (4) have known and identifiable positions on the surface of the conductive body (2) and the second electrodes (5) are placed at unknown spatial locations on the surface of the conductive body (2).

9. The system according to any of the preceding claims, wherein the first electrodes (4) comprise landmark electrodes (LE), whose position is known or identifiable in a repeatable manner at definite spatial locations on the surface of the conductive body (2), by means of: anatomical landmarks or and/or points temporarily or permanently marked and/or biopotentials or bioimpedance or physiological signal measurements; and the second electrodes (5) comprise measurement electrodes (ME), which are to be localized by the system (1).

10. A method for the automatic localization of electrode spatial position on the surface of a conductive body (2), comprising:

in response to bipolar electrical current injections at first electrodes (4) on the surface of the conductive body (2), acquiring voltages measured at second electrodes (5) on the surface of the conductive body (2); using an electrical impedance model of the conductive body (2), simulating the electrical current injections at the first electrodes (4) and estimating, for each electrical current injection, a resulting body surface potential on the surface of the conductive body (2); performing a combined processing of the voltages measured at the second electrodes (5) and of the estimated body surface potential, in order to determine the locations of the second electrodes (5) on the surface of the conductive body (2), further comprising determining the location of a respective electrode (m) as the spatial position on the surface of the conductive body (2) that provides in the estimated body surface potential given by the electrical impedance model a modelled voltage ($\hat{u}_{i,m}$), which most closely corresponds to the voltage ($u_{i,m}$) measured at the respective electrode (m), considering all current injections, wherein determining the location comprises:

determining, for each current injection (i) and corresponding voltage ($u_{i,m}$) measured at a respective electrode (m), a localization function on the electrical impedance model that represents a group of possible locations corresponding to that measured voltage ($u_{i,m}$); and determining the location of the respective electrode (m) via a combined processing of all the corresponding localization functions obtained for all the current injections (i).

11. The method according to claim 10, comprising:

obtaining, for each current injection (i) an isopotential line on the electrical impedance model based on the corresponding voltage ($u_{i,m}$) measured at the respective electrode (m), the isopotential line being indicative of all possible locations on the surface of the conductive body (2) matching that measured voltage ($u_{i,m}$); and determining the location of the respective electrode (m) based on a weighted mean or average of the intersections of all isopotential lines obtained for all the current injections.

12. The method according to claim 11, comprising:

computing, for each current injection (i), a difference function ($\Delta\Phi_{i,m}(x,y,z)$) based on a difference between the voltage ($u_{i,m}$) measured at the respective electrode (m) and the modelled voltage $\hat{u}_{i,m}$ on the surface of the conductive body (2) estimated from the electrical impedance model;

determining, based on the difference function ($\Delta\Phi_{i,m}(x,y,z)$), a respective probability function ($f_T(\Delta\Phi_i(x,y,z))$) indicative of a probability of localization of the respective electrode (m) at any spatial location (x,y,z) on the surface of the conductive body (2);

computing an average localization probability by averaging of the probability functions ($f_T(\Delta\Phi_i(x,y,z))$) of all current injections; and

determining the location of the respective electrode (m) based on the computed average localization probability.

13. The method according to any of claims 10-12, comprising assigning to each localization function and each corresponding current injection (i) a respective weighting factor ($w_i$) in the combined processing, the weighting factor ($w_i$) regulating the individual contribution of each current injection (i) to the electrode localization and being indicative of a localization reliability associated with each current injection (i).

14. The method according to claim 13, comprising:

performing, for each current injection (i), a calibration procedure for correction of the measured voltages ($u_{i,m}$) via a calibration function ($Cal_i$) based on the modelled voltages ($\hat{u}_{i,m}$) estimated by the electrical impedance model;

determining a goodness of fit of each calibration function;

determining, based on the related goodness of fit, the weighting factor ($w_i$) which regulates the individual contribution of each current injection (i) to the electrode localization.

15. The method according to any of claims 10-14, comprising:

computing, for each injection (i), calibration parameters for correction of the measured voltages ($u_{i,m}$), by calibrating voltages ($u_{i,k}$) measured at a set of electrodes with known or identifiable positions against modelled voltages ($\hat{u}_{i,k}$) estimated from the electrical impedance model for the same set of electrodes with known or identifiable positions; and

correcting all measured voltages ($u_{i,m}$) for the related current injection (i) at any respective electrode (m) to obtain corresponding calibrated voltages ($\bar{u}_{i,m}$), by means of a calibration function ($Cal_i$) and the computed calibration parameters.

16. The method according to any of claims 10-15, wherein the first electrodes (4) have known and identifiable positions on the surface of the conductive body (2) and the second electrodes (5) are placed at unknown spatial locations on the surface of the conductive body (2).


**Patentansprüche**

1. System (1) zum automatischen Lokalisieren der räumlichen Position von Elektroden auf der Oberfläche eines leitfähigen Körpers (2), umfassend eine Verarbeitungseinheit (10), die konfiguriert ist, um:

als Reaktion auf bipolare elektrische Strominjektionen an ersten Elektroden (4) auf der Oberfläche des leitfähigen Körpers (2), Spannungen zu erfassen, die an zweiten Elektroden (5) auf der Oberfläche des leitfähigen Körpers (2) gemessen werden;

unter Verwendung eines elektrischen Impedanzmodells des leitfähigen Körpers (2), an dem die elektrischen Strominjektionen an den ersten Elektroden (4) simuliert werden, für jede elektrische Strominjektion ein resultierendes Körperoberflächenpotential auf dem leitfähigen Körper (2) zu schätzen;

eine kombinierte Verarbeitung der an den zweiten Elektroden (5) gemessenen Spannungen und des geschätzten Körperoberflächenpotentials durchzuführen, um die Standorte der zweiten Elektroden (5) auf der Oberfläche des leitfähigen Körpers (2) zu bestimmen;

wobei die Verarbeitungseinheit (10) so konfiguriert ist, dass sie den Standort einer jeweiligen Elektrode (m) als die räumliche Position auf der Oberfläche des leitfähigen Körpers (2) bestimmt, die in dem durch das elektrische Impedanzmodell gegebenen geschätzten Körperoberflächenpotential eine modellierte Spannung ($\hat{u}_{i,m}$) bereitstellt, die der an der jeweiligen Elektrode (m) gemessenen Spannung ($u_{i,m}$) unter Berücksichtigung aller Strominjektionen (i) am nächsten entspricht,
und wobei die Verarbeitungseinheit (10) konfiguriert ist, um:

für jede Strominjektion (i) und entsprechende, an der jeweiligen Elektrode (m) gemessene Spannung ($u_{i,m}$) eine Lokalisierungsfunktion auf dem elektrischen Impedanzmodell zu bestimmen, die eine Gruppe möglicher Standorte entsprechend dieser gemessenen Spannung ($u_{i,m}$) darstellt; und
den Standort der jeweiligen Elektrode (m) über eine kombinierte Verarbeitung aller entsprechenden Lokalisierungsfunktionen zu bestimmen, die für alle Strominjektionen (i) erhalten wurden.

2. System nach Anspruch 1, wobei die Verarbeitungseinheit (10) konfiguriert ist, um:

für jede Strominjektion (i) eine Isopotentiallinie auf dem elektrischen Impedanzmodell auf der Grundlage der entsprechenden Spannung ($u_{i,m}$), die an der jeweiligen Elektrode (m) gemessen wurde, zu erhalten, wobei die Isopotentiallinie alle möglichen Standorte auf der Oberfläche des leitfähigen Körpers (2) angibt, die mit dieser gemessenen Spannung ($u_{i,m}$) übereinstimmen; und
den Standort der jeweiligen Elektrode (m) auf der Grundlage eines gewichteten Mittelwerts oder Durchschnitts der Schnittpunkte aller Isopotentiallinien zu bestimmen, die für alle Strominjektionen erhalten wurden.

3. System nach Anspruch 2, wobei die Verarbeitungseinheit (10) konfiguriert ist, um:

für jede Strominjektion (i) eine Differenzfunktion ($\Delta\Phi_{i,m}(x,y,z)$) auf der Grundlage einer Differenz zwischen der entsprechenden, an der jeweiligen Elektrode (m) gemessenen Spannung ($u_{i,m}$) und der modellierten, aus dem elektrischen Impedanzmodell abgeleiteten Spannung $\hat{u}_{i,m}$ auf der Oberfläche des leitfähigen Körpers (2) zu berechnen;
auf der Grundlage der Differenzfunktion ($\Delta\Phi_i(x,y,z)$), eine jeweilige Wahrscheinlichkeitsfunktion ($f_T(\Delta\Phi_i(x,y,z))$) zu bestimmen, die eine Wahrscheinlichkeit der Lokalisierung der jeweiligen Elektrode (m) an einem beliebigen räumlichen Standort (x,y,z) auf der Oberfläche des leitfähigen Körpers (2) angibt;
eine durchschnittlichen Lokalisierungswahrscheinlichkeit durch Mittelwertbildung der Wahrscheinlichkeitsfunktionen ($f_T(\Delta\Phi_i(x,y,z))$) aller aktuellen Injektionen zu berechnen; und
den Standort der jeweiligen Elektrode (m) auf der Grundlage der berechneten durchschnittlichen Lokalisierungswahrscheinlichkeit zu bestimmen.

4. System nach einem der vorhergehenden Ansprüche, wobei die Verarbeitungseinheit (10) so konfiguriert ist, dass sie jeder Lokalisierungsfunktion und jeder entsprechenden Strominjektion (i) einen jeweiligen Gewichtungsfaktor ($w_i$) in der kombinierten Verarbeitung zuweist, wobei der Gewichtungsfaktor ($w_i$) den individuellen Beitrag jeder Strominjektion (i) zur Elektrodenlokalisierung regelt und eine mit jeder Strominjektion (i) assoziierte Lokalisierungszuverlässigkeit angibt.

5. System nach Anspruch 4, wobei die Verarbeitungseinheit (10) konfiguriert ist, um:

für jede Stromeinspeisung (i) eine Kalibrierungsprozedur zur Korrektur der gemessenen Spannungen ($u_{i,m}$) über eine Kalibrierungsfunktion ($Cal_i$) auf der Grundlage der durch das elektrische Impedanzmodell geschätzten modellierten Spannungen ($\hat{u}_{i,m}$) durchführen;
die Güte der Anpassung jeder Kalibrierungsfunktion ($Cal_i$) zu bestimmen;
auf der Grundlage der zugehörigen Güte der Anpassung den Gewichtungsfaktor ($w_i$) zu bestimmen, der den individuellen Beitrag jeder Strominjektion (i) zur Elektrodenlokalisierung regelt.

6. System nach einem der vorhergehenden Ansprüche, wobei die Verarbeitungseinheit (10) konfiguriert ist, um:

für jede Injektion (i), Kalibrierungsparametern zur Korrektur der gemessenen Spannungen ($u_{i,m}$) durch Kalibrieren von Spannungen ($u_{i,k}$), die an einem Satz von Elektroden mit bekannten oder identifizierbaren Positionen gemessen wurden, gegen modellierte Spannungen ($\hat{u}_{i,k}$), die aus dem elektrischen Impedanzmodell für denselben Satz von Elektroden mit bekannten oder identifizierbaren Positionen geschätzt wurden, zu berechnen; und
alle gemessenen Spannungen ($u_{i,m}$) für die zugehörige Strominjektion (i) an einer beliebigen jeweiligen Elektrode (m) zu korrigieren, um entsprechende kalibrierte Spannungen ($\bar{u}_{i,m}$), mittels einer Kalibrierungsfunktion

(Cal$_i$) und der berechneten Kalibrierungsparameter zu erhalten.

7. System nach einem der vorangehenden Ansprüche, wobei die Verarbeitungseinheit (10) ferner so konfiguriert ist, dass sie eine mit der räumlichen Lokalisierung jeder zweiten Elektrode (5) assoziierte Zuverlässigkeit bewertet, indem sie einen entsprechenden Lokalisierungszuverlässigkeitsindex (LRI) berechnet.

8. System nach einem der vorhergehenden Ansprüche, wobei die ersten Elektroden (4) bekannte und identifizierbare Positionen auf der Oberfläche des leitfähigen Körpers (2) aufweisen und die zweiten Elektroden (5) an unbekannten räumlichen Standorten auf der Oberfläche des leitfähigen Körpers (2) platziert sind.

9. System nach einem der vorhergehenden Ansprüche, wobei die ersten Elektroden (4) Markierungselektroden (LE) umfassen, deren Position an bestimmten räumlichen Standorten auf der Oberfläche des leitfähigen Körpers (2) bekannt oder wiederholbar identifizierbar ist, und zwar durch: anatomische Markierungen oder und/oder Punkte, die vorübergehend oder dauerhaft markiert sind und/oder Biopotentiale oder Bioimpedanz oder physiologische Signalmessungen; und die zweiten Elektroden (5) umfassen Messelektroden (ME), die durch das System (1) zu lokalisieren sind.

10. Verfahren zum automatischen Lokalisieren der räumlichen Position von Elektroden auf der Oberfläche eines leitfähigen Körpers (2), umfassend:

als Reaktion auf bipolare elektrische Strominjektionen an ersten Elektroden (4) auf der Oberfläche des leitfähigen Körpers (2), Erfassen der Spannungen, die an zweiten Elektroden (5) auf der Oberfläche des leitfähigen Körpers (2) gemessen werden;
unter Verwendung eines elektrischen Impedanzmodells des leitfähigen Körpers (2), Simulieren der elektrischen Strominjektionen an den ersten Elektroden (4) und Schätzen, für jede elektrische Strominjektion, eines resultierenden Körperoberflächenpotentials auf der Oberfläche des leitfähigen Körpers (2);
Durchführen einer kombinierten Verarbeitung der an den zweiten Elektroden (5) gemessenen Spannungen und des geschätzten Körperoberflächenpotentials, um die Standorte der zweiten Elektroden (5) auf der Oberfläche des leitfähigen Körpers (2) zu bestimmen,
ferner umfassend das Bestimmen des Standortes einer jeweiligen Elektrode (m) als die räumliche Position auf der Oberfläche des leitfähigen Körpers (2), die in dem durch das elektrische Impedanzmodell gegebenen geschätzten Körperoberflächenpotential eine modellierte Spannung ($\hat{u}_{i,m}$) bereitstellt, die der an der jeweiligen Elektrode (m) gemessenen Spannung ($u_{i,m}$) unter Berücksichtigung aller Strominjektionen am nächsten entspricht,
wobei das Bestimmen des Standorts Folgendes umfasst:

Bestimmen, für jede Strominjektion (i) und entsprechende, an einer jeweiligen Elektrode (m) gemessene Spannung ($u_{i,m}$) eine Lokalisierungsfunktion auf dem elektrischen Impedanzmodell, die eine Gruppe möglicher Standorte entsprechend dieser gemessenen Spannung ($u_{i,m}$) darstellt; und
Bestimmen des Standorts der jeweiligen Elektrode (m) über eine kombinierte Verarbeitung aller entsprechenden Lokalisierungsfunktionen, die für alle Strominjektionen (i) erhalten wurden.

11. Verfahren nach Anspruch 10, ferner umfassend:

Erhalten, für jede Strominjektion (i) eine Isopotentiallinie auf dem elektrischen Impedanzmodell auf der Grundlage der entsprechenden Spannung ($u_{i,m}$), die an der jeweiligen Elektrode (m) gemessen wurde, wobei die Isopotentiallinie alle möglichen Standorte auf der Oberfläche des leitfähigen Körpers (2) angibt, die mit dieser gemessenen Spannung ($u_{i,m}$) übereinstimmen; und
Bestimmen des Standorts der jeweiligen Elektrode (m) auf der Grundlage eines gewichteten Mittelwerts oder Durchschnitts der Schnittpunkte aller Isopotentiallinien, die für alle Strominjektionen ermittelt wurden.

12. Verfahren nach Anspruch 11, ferner umfassend:

Berechnen, für jede Strominjektion (i), einer Differenzfunktion ($\Delta\Phi_{i,m}(x,y,z)$) auf der Grundlage einer Differenz zwischen der an der jeweiligen Elektrode (m) gemessenen Spannung ($u_{i,m}$) und der modellierten, aus dem elektrischen Impedanzmodell geschätzten Spannung $\hat{u}_{i,m}$ auf der Oberfläche des leitfähigen Körpers (2);
Bestimmen, auf Grundlage der Differenzfunktion ($\Delta\Phi_{i,m}(x,y,z)$), einer jeweiligen Wahrscheinlichkeitsfunktion ($f_T(\Delta\Phi_i(x,y,z))$), die eine Wahrscheinlichkeit der Lokalisierung der jeweiligen Elektrode (m) an einem beliebigen räumlichen Standort (x,y,z) auf der Oberfläche des leitenden Körpers (2) angibt;

Berechnen einer durchschnittlichen Lokalisierungswahrscheinlichkeit durch Mittelwertbildung der Wahrscheinlichkeitsfunktionen ($f_T(\Delta\Phi_i(x,y,z))$) aller aktuellen Injektionen; und

Bestimmen des Standorts der jeweiligen Elektrode (m) auf der Grundlage der berechneten durchschnittlichen Lokalisierungswahrscheinlichkeit.

13. Verfahren nach einem der Ansprüche 10-12, umfassend das Zuweisen zu jeder Lokalisierungsfunktion jeder Lokalisierungsfunktion und jeder entsprechenden Strominjektion (i) von einem jeweiligen Gewichtungsfaktor ($w_i$) in der kombinierten Verarbeitung zuweist, wobei der Gewichtungsfaktor ($w_i$) den individuellen Beitrag jeder Strominjektion (i) zur Elektrodenlokalisierung regelt und eine mit jeder Strominjektion (i) assoziierte Lokalisierungszuverlässigkeit angibt.

14. Verfahren nach Anspruch 13, ferner umfassend:

Durchführen, für jede Strominjektion (i), einer Kalibrierungsprozedur zur Korrektur der gemessenen Spannungen ($u_{i,m}$) über eine Kalibrierungsfunktion (Cal$_i$) auf der Grundlage der durch das elektrische Impedanzmodell geschätzten modellierten Spannungen ($\hat{u}_{i,m}$);

Bestimmen einer Güte der Anpassung jeder Kalibrierungsfunktion;

Bestimmen, auf der Grundlage der zugehörigen Güte der Anpassung, des Gewichtungsfaktors ($w_i$), der den individuellen Beitrag jeder Strominjektion (i) zur Elektrodenlokalisierung regelt.

15. Verfahren nach einem der Ansprüche 10-14, umfassend:

Berechnen, für jede Injektion (i), von Kalibrierungsparametern zur Korrektur der gemessenen Spannungen ($u_{i,m}$) durch Kalibrieren von Spannungen ($u_{i,k}$), die an einem Satz von Elektroden mit bekannten oder identifizierbaren Positionen gemessen wurden, gegen modellierte Spannungen ($\hat{u}_{i,k}$), die aus dem elektrischen Impedanzmodell für denselben Satz von Elektroden mit bekannten oder identifizierbaren Positionen geschätzt wurden; und

Korrigieren aller gemessenen Spannungen ($u_{i,m}$) für die zugehörige Strominjektion (i) an einer beliebigen jeweiligen Elektrode (m), um entsprechende kalibrierte Spannungen ($\overline{u}_{i,m}$) mittels einer Kalibrierfunktion (Cal$_i$) und der berechneten Kalibrierungsparameter.

16. Verfahren nach einem der Ansprüche 10-15, wobei die ersten Elektroden (4) bekannte und identifizierbare Positionen auf der Oberfläche des leitfähigen Körpers (2) aufweisen und die zweiten Elektroden (5) an unbekannten räumlichen Standorten auf der Oberfläche des leitfähigen Körpers (2) platziert sind.

## Revendications

1. Système (1) pour la localisation automatique de la position spatiale d'une électrode sur la surface d'un corps conducteur (2), comprenant une unité de traitement (10) configurée pour :

en réponse à des injections de courant électrique bipolaire au niveau de premières électrodes (4) sur la surface du corps conducteur (2), acquérir des tensions mesurées au niveau de secondes électrodes (5) sur la surface du corps conducteur (2) ;

à l'aide d'un modèle d'impédance électrique du corps conducteur (2), où les injections de courant électrique au niveau des premières électrodes (4) sont simulées, estimer, pour chaque injection de courant électrique, un potentiel de surface du corps résultant sur le corps conducteur (2) ;

effectuer un traitement combiné des tensions mesurées au niveau des secondes électrodes (5) et du potentiel de surface du corps estimé, afin de déterminer les emplacements des secondes électrodes (5) sur la surface du corps conducteur (2) ;

dans lequel l'unité de traitement (10) est configurée pour déterminer l'emplacement d'une électrode respective (m) comme étant la position spatiale sur la surface du corps conducteur (2) qui fournit, dans le potentiel de surface du corps estimé donné par le modèle d'impédance électrique, une tension modélisée ($\hat{u}_{i,m}$) qui correspond le mieux à la tension ($u_{i,m}$) mesurée au niveau de l'électrode respective (m), en tenant compte de toutes les injections de courant (i),

et dans lequel l'unité de traitement (10) est configurée pour :

déterminer, pour chaque injection de courant (i) et tension correspondante ($u_{i,m}$) mesurée au niveau de l'électrode respective (m), une fonction de localisation sur le modèle d'impédance électrique qui représente un groupe d'emplacements possibles correspondant à cette tension mesurée ($u_{i,m}$) ; et
déterminer l'emplacement de l'électrode respective (m) par l'intermédiaire d'un traitement combiné de toutes les fonctions de localisation correspondantes obtenues pour toutes les injections de courant (i).

2. Système selon la revendication 1, dans lequel l'unité de traitement (10) est configurée pour :

obtenir, pour chaque injection de courant (i), une ligne isopotentielle sur le modèle d'impédance électrique en fonction de la tension correspondante ($u_{i,m}$) mesurée au niveau de l'électrode respective (m), la ligne isopotentielle étant indicative de tous les emplacements possibles sur la surface du corps conducteur (2) correspondant à cette tension mesurée ($u_{i,m}$) ; et
déterminer l'emplacement de l'électrode respective (m) sur la base d'une moyenne pondérée ou d'une moyenne des intersections de toutes les lignes isopotentielles obtenues pour toutes les injections de courant.

3. Système selon la revendication 2, dans lequel l'unité de traitement (10) est configurée pour :

calculer, pour chaque injection de courant (i), une fonction de différence ($\Delta\Phi_{i,m}(x,y,z)$) basée sur une différence entre la tension correspondante ($u_{i,m}$) mesurée au niveau de l'électrode respective (m) et la tension modélisée $\hat{u}_{i,m}$ sur la surface du corps conducteur (2) dérivée du modèle d'impédance électrique ;
déterminer, sur la base de la fonction de différence ($\Delta\Phi_i(x,y,z)$), une fonction de probabilité respective ($f_T(\Delta\Phi_i(x,y,z))$) indiquant une probabilité de localisation de l'électrode respective (m) à n'importe quel emplacement spatial (x,y,z) sur la surface du corps conducteur (2) ;
calculer une probabilité de localisation moyenne en faisant la moyenne des fonctions de probabilité ($f_T(\Delta\Phi_i(x,y,z))$) de toutes les injections de courant ; et
déterminer l'emplacement de l'électrode respective (m) sur la base de la probabilité moyenne de localisation calculée.

4. Système selon l'une quelconque des revendications précédentes, dans lequel l'unité de traitement (10) est configurée pour attribuer à chaque fonction de localisation et à chaque injection de courant correspondante (i) un facteur de pondération respectif ($w_i$) dans le traitement combiné, le facteur de pondération ($w_i$) régulant la contribution individuelle de chaque injection de courant (i) à la localisation de l'électrode et étant indicatif d'une fiabilité de localisation associée à chaque injection de courant (i).

5. Système selon la revendication 4, dans lequel l'unité de traitement (10) est configurée pour :

exécuter, pour chaque injection de courant (i), une procédure d'étalonnage pour la correction des tensions mesurées ($u_{i,m}$) par l'intermédiaire d'une fonction d'étalonnage ($Cal_i$) sur la base des tensions modélisées ($\hat{u}_{i,m}$) estimées par le modèle d'impédance électrique ;
déterminer la qualité de l'ajustement de chaque fonction d'étalonnage ($Cal_i$) ;
déterminer, sur la base de la qualité de l'ajustement, le facteur de pondération ($w_i$) qui régule la contribution individuelle de chaque injection de courant (i) à la localisation de l'électrode.

6. Système selon l'une quelconque des revendications précédentes, dans lequel l'unité de traitement (10) est configurée pour :

calculer, pour chaque injection (i), les paramètres d'étalonnage pour la correction des tensions mesurées ($u_{i,m}$), en étalonnant les tensions ($u_{i,k}$) mesurées au niveau d'un ensemble d'électrodes dont les positions sont connues ou identifiables par rapport aux tensions modélisées ($\hat{u}_{i,k}$) estimées à partir du modèle d'impédance électrique pour le même ensemble d'électrodes dont les positions sont connues ou identifiables ; et
corriger toutes les tensions mesurées ($u_{i,m}$) pour l'injection de courant correspondante (i) au niveau de toute électrode respective (m) afin d'obtenir des tensions étalonnées correspondantes ($\overline{u}_{i,m}$), au moyen d'une fonction d'étalonnage ($Cal_i$) et des paramètres d'étalonnage calculés.

7. Système selon l'une quelconque des revendications précédentes, dans lequel l'unité de traitement (10) est en outre configurée pour évaluer une fiabilité associée à la localisation spatiale de chaque seconde électrode (5), en calculant un indice de fiabilité de la localisation (LRI) correspondant.

8. Système selon l'une quelconque des revendications précédentes, dans lequel les premières électrodes (4) ont des positions connues et identifiables sur la surface du corps conducteur (2) et les secondes électrodes (5) sont placées à des emplacements spatiaux inconnus sur la surface du corps conducteur (2).

9. Système selon l'une quelconque des revendications précédentes, dans lequel les premières électrodes (4) comprennent des électrodes repères (LE), dont la position est connue ou identifiable de manière reproductible à des emplacements spatiaux définis sur la surface du corps conducteur (2), au moyen de : repères anatomiques ou et/ou de points marqués de manière temporaire ou permanente et/ou de mesures de biopotentiels ou de bioimpédance ou de signaux physiologiques ; et les secondes électrodes (5) comprennent des électrodes de mesure (ME), qui doivent être localisées par le système (1).

10. Procédé de localisation automatique de la position spatiale d'une électrode sur la surface d'un corps conducteur (2), comprenant :

en réponse à des injections de courant électrique bipolaire au niveau de premières électrodes (4) sur la surface du corps conducteur (2), l'acquisition de tensions mesurées au niveau de secondes électrodes (5) sur la surface du corps conducteur (2) ;
à l'aide d'un modèle d'impédance électrique du corps conducteur (2), la simulation des injections de courant électrique au niveau des premières électrodes (4) et l'estimation, pour chaque injection de courant électrique, d'un potentiel de surface du corps résultant sur la surface du corps conducteur (2) ;
la réalisation d'un traitement combiné des tensions mesurées au niveau des secondes électrodes (5) et du potentiel de surface du corps estimé, afin de déterminer les emplacements des secondes électrodes (5) sur la surface du corps conducteur (2),
comprenant en outre la détermination de l'emplacement d'une électrode respective (m) comme étant la position spatiale sur la surface du corps conducteur (2) qui fournit dans le potentiel de surface du corps estimé donné par le modèle d'impédance électrique une tension modélisée ($\hat{u}_{i,m}$), qui correspond le plus étroitement à la tension ($u_{i,m}$) mesurée à l'électrode respective (m), en tenant compte de toutes les injections de courant,
dans lequel la détermination de l'emplacement comprend :

la détermination, pour chaque injection de courant (i) et tension correspondante ($u_{i,m}$) mesurée au niveau d'une électrode respective (m), d'une fonction de localisation sur le modèle d'impédance électrique qui représente un groupe d'emplacements possibles correspondant à cette tension mesurée ($u_{i,m}$) ; et
la détermination de l'emplacement de l'électrode respective (m) par l'intermédiaire d'un traitement combiné de toutes les fonctions de localisation correspondantes obtenues pour toutes les injections de courant (i).

11. Procédé selon la revendication 10, comprenant :

l'obtention, pour chaque injection de courant (i), d'une ligne isopotentielle sur le modèle d'impédance électrique en fonction de la tension correspondante ($u_{i,m}$) mesurée au niveau de l'électrode respective (m), la ligne isopotentielle étant indicative de tous les emplacements possibles sur la surface du corps conducteur (2) correspondant à cette tension mesurée ($u_{i,m}$) ; et
la détermination de l'emplacement de l'électrode respective (m) sur la base d'une moyenne pondérée ou d'une moyenne des intersections de toutes les lignes isopotentielles obtenues pour toutes les injections de courant.

12. Procédé selon la revendication 11, comprenant :

le calcul, pour chaque injection de courant (i), d'une fonction de différence ($\Delta\Phi_{i,m}(x,y,z)$) basée sur une différence entre la tension ($u_{i,m}$) mesurée au niveau de l'électrode respective (m) et la tension modélisée $\hat{u}_{i,m}$ sur la surface du corps conducteur (2) estimée à partir du modèle d'impédance électrique ;
la détermination, sur la base de la fonction de différence ($\Delta\Phi_i(x,y,z)$), d'une fonction de probabilité respective ($f_T(\Delta\Phi_i(x,y,z))$) indiquant une probabilité de localisation de l'électrode respective (m) à n'importe quel emplacement spatial (x,y,z) sur la surface du corps conducteur (2) ;
le calcul d'une probabilité de localisation moyenne en faisant la moyenne des fonctions de probabilité ($f_T(\Delta\Phi_i(x,y,z))$) de toutes les injections de courant ; et
la détermination de l'emplacement de l'électrode respective (m) sur la base de la probabilité moyenne de localisation calculée.

13. Procédé selon l'une quelconque des revendications 10 à 12, comprenant l'attribution à chaque fonction de localisation et à chaque injection de courant correspondante (i) d'un facteur de pondération respectif ($w_i$) dans le

traitement combiné, le facteur de pondération ($w_i$) régissant la contribution individuelle de chaque injection de courant (i) à la localisation de l'électrode et étant indicatif d'une fiabilité de localisation associée à chaque injection de courant (i).

14. Procédé selon la revendication 13, comprenant :

l'exécution, pour chaque injection de courant (i), d'une procédure d'étalonnage pour la correction des tensions mesurées ($u_{i,m}$) par l'intermédiaire d'une fonction d'étalonnage (Cali) basée sur les tensions modélisées ($\hat{u}_{i,m}$) estimées par le modèle d'impédance électrique ;
la détermination de la qualité de l'ajustement de chaque fonction d'étalonnage ;
la détermination, sur la base de la qualité de l'ajustement, du facteur de pondération ($w_i$) qui régule la contribution individuelle de chaque injection de courant (i) à la localisation de l'électrode.

15. Procédé selon l'une quelconque des revendications 10 à 14, comprenant :

le calcul, pour chaque injection (i), des paramètres d'étalonnage pour la correction des tensions mesurées ($u_{i,m}$), en étalonnant les tensions ($u_{i,k}$) mesurées à un ensemble d'électrodes dont les positions sont connues ou identifiables par rapport aux tensions modélisées ($\hat{u}_{i,k}$) estimées à partir du modèle d'impédance électrique pour le même ensemble d'électrodes dont les positions sont connues ou identifiables ; et
la correction de toutes les tensions mesurées ($u_{i,m}$) pour l'injection de courant correspondante (i) au niveau de toute électrode respective (m) afin d'obtenir des tensions étalonnées correspondantes ($\overline{u}_{i,m}$), au moyen d'une fonction d'étalonnage (Cal$_i$) et des paramètres d'étalonnage calculés.

16. Procédé selon l'une quelconque des revendications 10 à 15, dans lequel les premières électrodes (4) ont des positions connues et identifiables sur la surface du corps conducteur (2) et les secondes électrodes (5) sont placées à des emplacements spatiaux inconnus sur la surface du corps conducteur (2).

FIG.1

100

Current injection 6

Processing unit 10

Voltage measurement 8

1

2

4

5

FIG.2

FIG.3

ISOPOTENTIAL
LINES

FIG.4

FIG.5A

FIG.5B

Ventral View    Left View    Dorsal View    Right View

FIG.6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- RU 2733470 C1
- WO 2019235969 A1
- WO 2018218244 A1
- WO 2011159688 A2
- US 5297549 A
- WO 2020212527 A1

### Non-patent literature cited in the description

- **E. A. PEREZ-ALDAY et al.** Torso geometry reconstruction and body surface electrode localization using three-dimensional photography. *Journal of Electrocardiology*, January 2018, vol. 51 (1), 60-67
- **E. A. PEREZ-ALDAY et al.** Torso mesh and body surface electrodes position reconstruction using a Kinect camera. *Journal of Electrocardiology*, January 2018, vol. 51 (1), e4-e5
- **R. N. GHANEM ; C. RAMANATHAN ; PING JIA ; Y. RUDY**. Heart-surface reconstruction and ECG electrodes localization using fluoroscopy, epipolar geometry and stereovision: application to noninvasive imaging of cardiac electrical activity. *IEEE Transactions on Medical Imaging*, October 2003, vol. 22 (10), 1307-1318
- **LENCHIK, LEON et al.** Automated segmentation of tissues using CT and MRI: a systematic review. *Academic radiology*, 2019, vol. 26 (12), 1695-1706
- **A. PEREZ-ALDAY et al.** Torso geometry reconstruction and body surface electrode localization using three-dimensional photography. *Journal of Electrocardiology*, January 2018, vol. 51 (1), 60-67
- **A. PEREZ-ALDAY et al.** Torso mesh and body surface electrodes position reconstruction using a Kinect camera. *Journal of Electrocardiology*, January 2018, vol. 51 (1), e4-e5
- Electrical impedance tomography: methods, history, and applications. Institute of Physics Publishing, 2005